(19) Europäisches Patentamt / European Patent Office / Office européen des brevets

(11) **EP 2 524 235 B1**

(12) **EUROPEAN PATENT SPECIFICATION**

(45) Date of publication and mention of the grant of the patent:
**21.05.2014 Bulletin 2014/21**

(51) Int Cl.:
*G01R 31/12* (2006.01)     *G01N 33/28* (2006.01)

(21) Application number: **11705253.0**

(22) Date of filing: **10.01.2011**

(86) International application number:
**PCT/IB2011/050081**

(87) International publication number:
**WO 2011/086482 (21.07.2011 Gazette 2011/29)**

(54) **DIAGNOSTIC METHOD AND APPARATUS FOR ASSESSING THE INSULATION CONDITION OF ELECTRICAL EQUIPMENT INSULATED WITH OIL**

DIAGNOSEVERFAHREN UND -VORRICHTUNG ZUM ÜBERPRÜFEN DES ISOLATIONSZUSTANDES EINER MIT ÖL ISOLIERTEN ELEKTRISCHEN VORRICHTUNG

PROCÉDÉ DE DIAGNOSTIC ET APPAREIL PERMETTANT D'ÉVALUER L'ÉTAT D'ISOLATION D'UN ÉQUIPEMENT ÉLECTRIQUE ISOLÉ PAR DE L'HUILE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.01.2010 IT BO20100017**

(43) Date of publication of application:
**21.11.2012 Bulletin 2012/47**

(73) Proprietor: **Techimp Technologies S.r.l.**
**40121 Bologna (IT)**

(72) Inventors:
• **CAVALLINI, Andrea**
  **40121 Bologna (IT)**
• **CIANI, Fabio**
  **40121 Bologna (IT)**
• **SERRA, Stefano**
  **40121 Bologna (IT)**
• **MONTANARI, Gian Carlo**
  **40121 Bologna (IT)**

(74) Representative: **Conti, Marco**
  **Bugnion S.p.A.**
  **Via di Corticella, 87**
  **IT-40128 Bologna (IT)**

(56) References cited:
**EP-A1- 1 637 879     WO-A1-03/075294**
**US-A- 4 654 806     US-B1- 6 289 716**

• HARRIS D ET AL: "Condition monitoring in power transformers", 19970619, 19 June 1997 (1997-06-19), pages 7/1-7/3, XP006509897,
• DUVAL M: "The duval triangle for load tap changers, non-mineral oils and low temperature faults in transformers", IEEE ELECTRICAL INSULATION MAGAZINE, IEEE SERVICE CENTER, NEW YORK, NY, US LNKD- DOI: 10.1109/MEI.2008.4665347, vol. 11, no. 6, 1 November 2008 (2008-11-01), pages 22-29, XP011266847, ISSN: 0883-7554
• DHINGRA ARVIND ET AL: "Condition monitoring of power transformer: A review", TRANSMISSION AND DISTRIBUTION CONFERENCE AND EXPOSITION, 2008. T&D. IEEE/PES, IEEE, PISCATAWAY, NJ, USA, 21 April 2008 (2008-04-21), pages 1-6, XP031250069, ISBN: 978-1-4244-1903-6
• WENZEL D ET AL: "Partial discharge measurement and gas monitoring of a power transformer on-site", 23 September 1996 (1996-09-23), DIELECTRIC MATERIALS, MEASUREMENTS AND APPLICATIONS, SEVENTH INTERNATI ONAL CONFERENCE ON (CONF. PUBL. NO. 430) BATH, UK 23-26 SEPT. 1996, LONDON, UK,IEE, UK LNKD- DOI: 10.1049/CP:19961033, PAGE(S) 255 - 258, XP006507887, ISBN: 978-0-85296-670-9 page 258, column 1, line 13 - line 28 page 258, column 2, line 1 - line 12

EP 2 524 235 B1

- DURAISAMY ET AL: "Neuro fuzzy schemes for fault detection in power transformer", APPLIED SOFT COMPUTING, ELSEVIER LNKD- DOI: 10.1016/J.ASOC.2006.10.001, vol. 7, no. 2, 7 February 2007 (2007-02-07), pages 534-539, XP005877623, ISSN: 1568-4946
- NADERIAN A ET AL: "An Approach to Determine the Health Index of Power Transformers", 9 June 2008 (2008-06-09), ELECTRICAL INSULATION, 2008. ISEI 2008. CONFERENCE RECORD OF THE 2008 IEEE INTERNATIONAL SYMPOSIUM ON, IEEE, PISCATAWAY, NJ, USA, PAGE(S) 192 - 196, XP031289155, ISBN: 978-1-4244-2091-9 the whole document
- ARSHAD M ET AL: "Power transformer insulation response and risk assessment", 12 September 2004 (2004-09-12), PROBABILISTICMETHODS APPLIED TO POWER SYSTEMS, 2004 INTERNATIONAL CONF ERENCE ON AMES, IA, USA 12-16 SEPT. 2004, PISCATAWAY, NJ, USA, IEEE, PAGE(S) 502 - 505, XP010758112, ISBN: 978-0-9761319-1-5 the whole document

**Description**

Technical Field

This invention relates to a diagnostic method and apparatus for assessing the insulation condition of electrical equipment insulated with oil.

More specifically, the invention addresses the field of diagnostic assessment of electrical transformers insulated with oil. In effect, in the field of medium- or high-voltage transformers, oil is frequently used to insulate the transformers.

Background Art

Diagnostic apparatuses for assessing the insulation condition of transformers (or other medium- or high-voltage equipment) insulated with oil have been in use for some time. These apparatuses are based on a technique known as DGA (dissolved gas analysis), for estimating the concentration of gases dissolved in the oil.

DGA is based on measuring the concentration of predetermined types of gases dissolved in the insulation oil in order to derive a diagnostic indication about the insulation condition of the transformer, that is to say, to derive an indication about the source which generates gas in the oil.

More specifically, DGA is used to analyse the concentration of a plurality of gases, including hydrogen, methane, ethane, ethylene, acetylene, carbon monoxide and carbon dioxide.

The umbrella term DGA encompasses several prior art techniques, such as, for example, the Duval triangle method or the techniques based on IEC 60599.

The use of different prior art DGA methods for diagnosing the insulation condition of a transformer starting from the same measured concentration values of gases in the oil often give conflicting diagnostic indications, depending on the specific techniques used. In effect, the techniques often conflict (in terms of criteria for interpreting the data collected for the purpose of identifying the source of the gases detected in the oil).

Thus, a first disadvantage of known diagnostic apparatuses and DGA diagnostic methods is that they often lead to relatively unreliable indications.

In light of this, it should be noted that the reliability of diagnostic indications for transformers, and more generally, for any electrical equipment, is of crucial importance because technical personnel use these indications to programme maintenance schedules and/or for prompt action to restore optimum insulation conditions of the electrical equipment diagnosed.

A second disadvantage of prior art diagnostic apparatuses based on DGA is connected with the need to identify the sources of the gases dissolved in the oil and to distinguish between the different possible sources in a reliable manner.

As a matter of fact, with the prior art DGA techniques, only a very small number of source types can be identified easily and reliably.

In effect, for identifying many types of sources, the prior art techniques involve detecting types of gases present in very small concentrations. That means these techniques are less reliable, making the gas detection systems based on them more complicated and expensive.

Thus, DGA based diagnostic apparatuses can provide diagnostic indications which can be used to identify only a few sources (causes of degradation) that lead to the formation of gases in a transformer.

Moreover, it should be noted that diagnostic apparatuses based on more advanced DGA techniques involve estimating the concentration of the gas in the oil to be used in subsequent DGA as a function of the value measured inside a measuring chamber separated from the oil container by a membrane permeable to the gas.

The measuring chamber receives through the membrane a part of the gas present in the oil.

Diagnostic apparatuses of this kind are equipped with one or more sensors inside the measuring chamber to measure the concentration of the gas in the measuring chamber itself.

An erroneous estimate of the concentration of a gas dissolved in the oil nullifies the result of subsequent DGA, which consequently provides an incorrect diagnostic indication or, in the worst of cases, does not detect a possible source of degradation in the insulation of the electrical equipment.

This poses a problem of correctly estimating the gas concentration in the oil as a function of the amount of gas measured in the measuring chamber. In effect, it takes a relatively long time for the gas to pass from the oil to the measuring chamber (transient of permeation through the membrane) and this leads to errors estimating the concentration.

Thus, another disadvantage of these diagnostic apparatuses is that they are not robust and are subject to gross errors, since there is a real risk of the estimated gas concentration (used in the known diagnostic methods) being wrong (especially during the transients following the formation of the gas in the oil).

It should be noted that the gas present in the oil is produced (usually) by electrical discharges, also known as partial discharges, which occur in the insulation oil or in other parts of the insulation of the electrical equipment.

Partial discharge is a well-known phenomenon in electrical equipment subjected to medium or high voltages.

A partial discharge is an electric discharge limited to a portion of the insulation of an electrical system and does not therefore cause immediate failure of the system but, more generally, causes its gradual degradation.

By their very nature, therefore, partial discharges are substantially limited in extent to a defect in the insulating system.

It should be noted that partial discharge signals are measured and analysed for diagnostic purposes in the case of electrical equipment with solid or gaseous insulation.

In the case of electrical equipment insulated with oil, however, partial discharge analysis (PDA) methods are not used.

In effect, oil insulation is self-restorative, which means that the defects (partial discharge sources) typical of oil insulators are subject to change and even disappear over time.

To this must be added the fact that partial discharges measured on transformers insulated with oil are significantly affected by noise and do not allow use of interpretation methods normally used on equipment with solid insulators

Patent document US4654806 discloses a transformer monitoring system wherein parameters pertaining to the DGA are used in combination with a parameter pertaining to PD.

The following scientific articles
'Harris D et al: "conditioning monitoring in power transformers", 19 June 1997', discloses the possibility to use a combination of different monitoring systems for diagnostic assessment of a transformer, among which PD and DGA.

The scientific article 'Duval M: "the duval triangle for load tap changers, non-mineral oils and low temperature faults in transformers", IEEE electrical insulation magazine, Vol. 11 n. 6, 1 Nov. 2008', and

'Dhingra Arvind et al: "Condition monitoring of power transformer: a review", Transmission and distribution conference and exposition, T&D IEEE, USA 21 April 2008'
disclose interpretation strategies for the DGA results, with the aim of identifying lists of fault sources for an oil insulated transformer.

Furthermore, the scientific article 'Wenzel D et al :" Partial discharge measurement and gas monitoring of a power transformer on-site", 7th international conference on dielectric materials, measurements and applications, UK, 23.September 1996, discloses that PD signals can be processed by calculating phase and amplitude parameters and discusses internal, external and corona partial discharge categories.

Aim of the Invention

This invention has for an aim to provide a diagnostic method and apparatus for assessing the insulation condition of electrical equipment insulated with oil and which overcome the above mentioned disadvantages of the prior art.

In particular, the invention has for an aim to provide a diagnostic method and apparatus for assessing the insulation condition of electrical equipment insulated with oil and which can provide reliable and accurate information about the insulation condition of the electrical equipment itself.

Another aim of the invention is to provide a diagnostic method and apparatus for assessing the insulation condition of a transformer insulated with oil and which can identify in a simple and reliable manner a large number of defects in the insulation of the transformer itself.

These aims are fully achieved by the method and apparatus according to the invention as characterized in the appended independent claims 1 and 15.

More specifically, the method of this invention comprises the following steps:

- measuring the concentration of a gas dissolved in the insulating oil of the electrical equipment;
- deriving at least one concentration parameter correlated with the gas concentration measured in a predetermined acquisition time interval.

The method further comprises the following steps:

- measuring electrical pulses relating to partial electrical discharges which occur in the electrical equipment and which generate said pulses;
- deriving at least one discharge parameter correlated with the partial discharges measured substantially concurrently with the predetermined acquisition time interval;
- deriving a diagnostic indication about the insulation condition of the electrical equipment as a function of the derived values of the concentration and discharge parameters.

The apparatus of the invention is equipped with a device for measuring the concentration of a gas dissolved in the insulating oil of the electrical equipment.

The apparatus comprises, combined together: a module for measuring electrical pulses relating to partial electrical discharges which occur in the electrical equipment and which generate said pulses; a processing unit connected to the device and to the module for measuring the partial discharges and designed to derive at least one concentration parameter correlated with the measured gas concentration and at least one discharge parameter correlated with the partial discharges and to derive a diagnostic indication about the insulation condition of the electrical equipment as a function of the derived values of the concentration parameter and of the derived values of the discharge parameter, in combination.

Preferably, this combination of the (values of the) concentration and discharge parameters is a non-linear combination.

Brief Description of the Drawings

These and other features of the invention will become more apparent from the following description of a preferred, non-limiting embodiment of it, with reference to the accompanying drawings, in which:

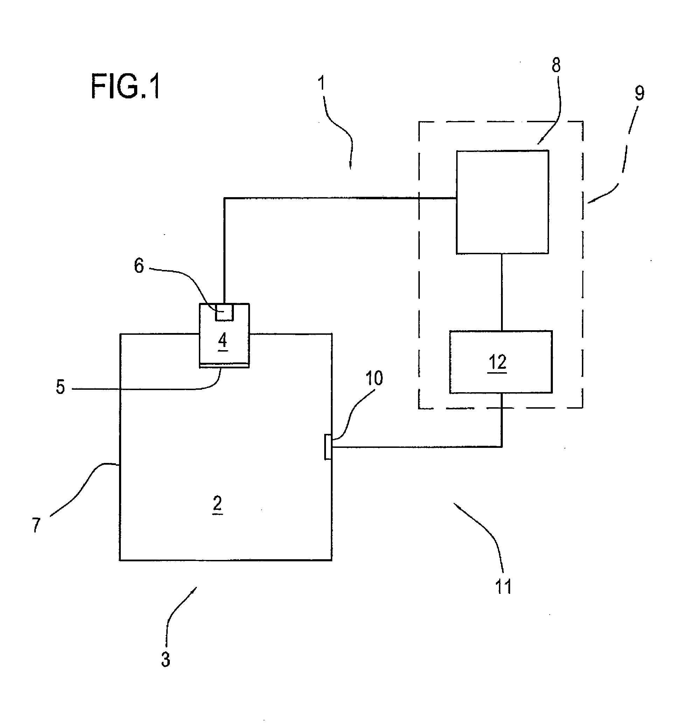

- Figure 1 schematically illustrates a device according to this invention;
- Figure 2 shows a flow chart representing the method according to this invention.

Detailed Description of the Preferred Embodiments of the Invention

In the drawings. the numeral 11 denotes a diagnostic apparatus for assessing the insulation condition of electrical equipment 3 insulated with oil 2.

Generally speaking, the electrical equipment 3 is any electrical equipment (for medium or high voltages) insulated with oil, such as, for example, a transformer, a cable or a switch.

In particular, however, this invention addresses a diagnostic apparatus for assessing the insulation condition of a transformer insulated with oil.

Therefore, in the description which follows, the equipment 3 is a transformer.

This shall not, however, be construed as a limitation of the scope of this invention as the diagnostic apparatus 11 can be associated with other types of electrical equipment, such as, for example, a cable or any electrical equipment insulated with oil.

The oil 2 of the transformer is held in a container 7, hereinafter referred to as container 7 of the oil 2.

The diagnostic apparatus 11 is equipped with a device 1 for measuring the concentration of gases dissolved in the oil.

In a preferred embodiment, the device 1 comprises a membrane 5, permeable to gas and interposed between the container 7 of the oil 2 and a measuring chamber 4 to allow gases to pass through it from the container 7 of the oil to the measuring chamber 4.

The device 1 also comprises a sensor 6 mounted in the measuring chamber 4, for measuring the concentration of the gases in the measuring chamber 4, and a control unit 8.

The sensor 6 can measure the concentration of one or more predetermined types of gas.

More specifically, the device 1 is configured to measure at least one of the gases listed below:

- carbon monoxide, hereinafter also referred to as CO;
- hydrogen, hereinafter also referred to as $H_2$.

Preferably, the device 1 is configured to measure both of the gases listed above.

Alternatively, instead of a single sensor 6, the device 1 might comprise a plurality of sensors, each designed to measure the concentration of a predetermined type of gas.

The device 1 comprises a control unit 8 (or a processor or any other processing means) electrically connected to the sensor 6 to receive from the latter a signal corresponding to the value/values of concentration of the predetermined type/types of gas measured in the measuring chamber 4.

The control unit 8 comprises, preferably, but without limiting the scope of the invention, a memorization module (not illustrated) and a processing module (also not illustrated) functionally connected to the memorization module.

The control unit 8 defines processing means 9 configured to derive an estimated value of gas concentration in the oil 2, as a function of a corresponding value of gas concentration measured inside the measuring chamber 4.

Preferably, the device 1 also comprises a timer connected to the control unit 8 and designed to generate a signal which can be used by the processing module of the control unit 8 to generate (and memorize) measuring instants corresponding to the measurements taken by the sensor 6 in succession. The timer is connected to the control unit 8 also to allow a plurality of measurements (of the value of gas concentration in the measuring chamber 4) to be taken in succession at predetermined measuring instants.

The memorization module of the control unit 8 is designed to memorize the gas concentration values acquired by the sensor 6.

The control unit 8 associates a time information item, according to known techniques, with each acquired value of gas concentration in the measuring chamber 4, obtained, for example, by the timer and relating to the acquisition instant at which that gas concentration value is acquired.

For example, for each gas concentration value measured in the measuring chamber 4, the control unit 8 can memorize directly in the memorization module the time information item regarding the instant that value is acquired; and/or can sort the acquired values of gas concentration in the measuring chamber 4 according to a predetermined sequence and use a predetermined sampling step.

Described below is the operation of the device 1 for deriving the concentration of a gas dissolved in electrical insulation oil 2.

Hereinafter, the following notation will be used:

- $t_i$ to denote a time instant;
- $X_i$ to denote the value for the concentration of a predetermined gas inside the measuring chamber 4 measured by the sensor 6 at the time instant $t_i$;
- $Y_i$ to denote the estimated value of gas concentration in the oil, calculated by the control unit 8;
- $\bar{t}$ to denote a predetermined time interval;
- $\bar{x}$ to denote a predetermined interval of variation of the gas concentration in the measuring chamber 4 preferably in the predetermined time interval $\bar{t}$;
- K to denote the measurements taken (in the measuring time interval, not less than $\bar{t}$).

Express reference will hereinafter be made to the measurement of the concentration of a generic gas type inside the measuring chamber 4.

The method proposed can therefore be used to measure the concentration of any gas (or plurality of gases) inside the measuring chamber 4 and to estimate its concentration in the oil 2 accordingly.

The electronic control unit 8 acquires from the sensor 6, in a predetermined time interval T (where T is not less than $\bar{t}$), a plurality of values $(X_1, X_2,...,X_k)$ for the concentration of one predetermined type of gas inside the measuring chamber 4.

Preferably, the predetermined time interval $\bar{t}$ is approximately 24 hours.

Preferably, the concentration values $(X_1, X_2,...,X_k)$ measured inside the measuring chamber 4 are acquired at predetermined time intervals.

More specifically, preferably, but without limiting the scope of the invention, the gas concentration values $(X_1, X_2,...,X_k)$ measured inside the measuring chamber 4 are spaced at a constant time interval, that is to say, these concentration values $(X_1, X_2,...,X_k)$ are acquired by the control unit 8 preferably with a constant sampling step.

This advantageously simplifies subsequent processing of the measured concentration values by the control unit 8.

The concentration values $(X_1, X_2,...,X_k)$ measured inside the measuring chamber 4 are spaced preferably at a time interval of 15-25 minutes, and still more preferably, approximately twenty minutes.

According to the invention, however, the concentration values $(X_1, X_2,...,X_k)$ measured inside the measuring chamber 4 might also be spaced at non-constant time intervals, that is to say, these concentration values $(X_1, X_2,...,X_k)$ might be acquired by the control unit 8 with a sampling step that is not constant.

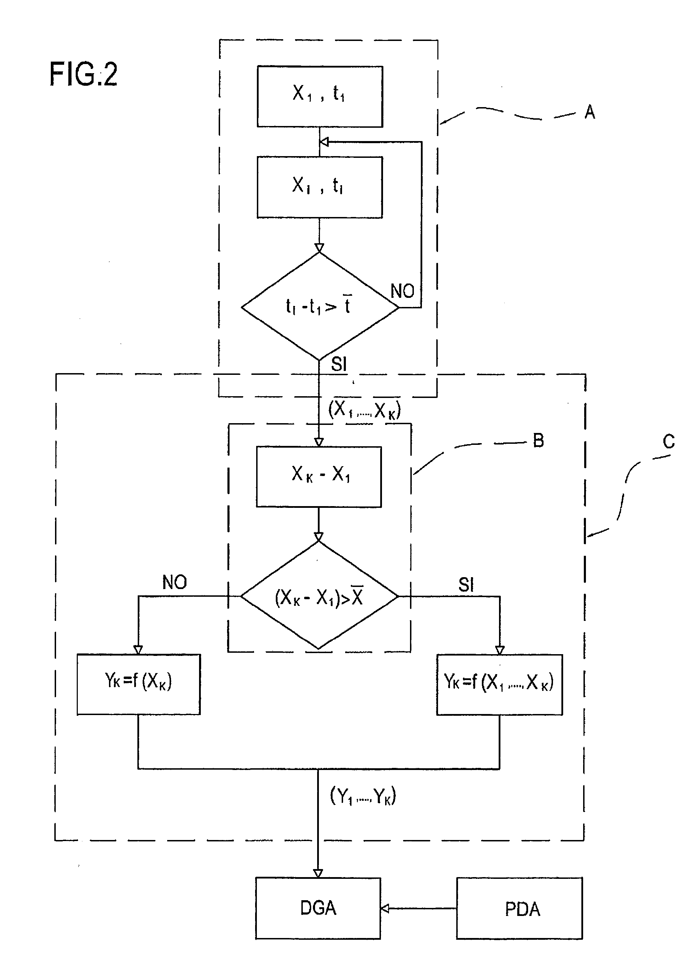

At each acquisition of a concentration value $X_i$ in the measuring chamber 4, the control unit 8 checks whether $(t_i - t_1) > \bar{t}$, that is to say, whether or not the predetermined time interval $\bar{t}$ has passed from the time the first sample $X_1$ was acquired, as illustrated in the block A of the schematic diagram of Figure 2.

It should therefore be observed that the acquisition time interval T, equal to $t_k - t_l$, is not less than the predetermined time interval $\bar{t}$.

The first value acquired after the period $\bar{t}$, $X_k$, is compared, preferably but without limiting the scope of the invention, with the very first value acquired, $X_1$.

Alternatively, the first value acquired after the predetermined time interval $\bar{t}$ might be compared with one or more of the previously acquired values $X_1 \div X_k$-1.

This comparison is a comparison of the threshold type, that is to say, the difference $(X_k-X_1)$ is compared with a predetermined interval $\bar{x}$ of variation of the gas concentration in the measuring chamber 4.

If the difference $(X_k-X_1)$ is greater than the predetermined interval $\bar{x}$ of variation of the gas concentration in the measuring chamber 4, the estimated value $Y_k$ for the gas concentration in the oil corresponding to the value $X_k$ is derived by means of a non-linear function of the value $X_k$ and of the gas concentration values $(X_1, X_2,...,X_{k-1})$ previously measured

in the measuring chamber 4, that is to say, a non-linear function of the $Y_k=f(X_1,X_2,......X_k)$ type.

It should be noted that this check is also carried out for any other value $X_i$ acquired after the first ($X_1$), as described above.

In effect, exceeding the predetermined interval $\bar{x}$ of variation of the gas concentration in the measuring chamber 4 indicates that a more or less sudden variation in the gas concentration in the oil 2 of the electrical equipment 3 is in progress and, hence, that a transient of gas transfer through the membrane 5 is in progress.

Under these conditions it is very likely that an equilibrium has not yet been reached between the gas concentration in the oil 2 and the gas concentration inside the measuring chamber 4, on account of the dynamics of the phenomenon by which the gas passes through the membrane 5 from the oil 2 to the measuring chamber 4.

This non-linear function is also used to estimate the gas concentration in the oil for all the concentration values ($X_1$, $X_2$,....) measured after the first (after finding that the difference ($X_i$-$X_1$) is greater than the predetermined value $\bar{x}$).

Preferably, the non-linear function (which links a predetermined gas concentration measured in the chamber 4 to the corresponding concentration of the same gas in the oil 2 in the container 7) is the function shown below by way of an example for the gas concentration value $X_k$ acquired in the measuring chamber 4.

$$Yk = \frac{(X_k - X_1 e^{-R_d t_k})}{R_d \lambda(T_g,P)\,erfc(\frac{d}{\sqrt{4D_i t_k}})} - \frac{e^{-R_d t_k}\int^{t_{k-1}} dt' \, X(t')erfc(\frac{d}{\sqrt{4D_i t'}})e^{R_d t'}}{erfc(\frac{d}{\sqrt{4D_i t_i}})}$$

Where:

- $\lambda(T_g, P)$ is Ostwald's solubility coefficient, which is a function of temperature and pressure,

- $erfc(\frac{d}{\sqrt{4D_i t}})$ is the complementary error function, and

- Rd and Di are experimental constants calculated on the basis of the polymer the membrane is made of.

The estimated value of gas concentration in the oil, $Y_k$, is calculated by the control unit 8, and more specifically, by the processing module.

Advantageously, the aforesaid non-linear function takes into account:

- the dynamics of the process of gas diffusion through the membrane 5, this diffusion process being relatively slow;
- the process of absorption and de-absorption of the gas through the membrane 5.

The aforesaid non-linear function therefore takes into account the transient of gas permeation through the membrane 5 in the predetermined time interval $\bar{t}$.

Thus, the device 1 advantageously makes it possible to derive, with a high degree of accuracy, the value of gas concentration in the oil 2; more specifically, the device 1 makes it possible to obtain a good estimation of the gas concentration in the oil 2 even for relatively slow gas-oil system transients.

Further, advantageously, the device 1 does not require complex calibrations to correlate the value $X_k$ (that is, any measured value $X_i$) of gas concentration inside the measuring chamber 4 with the value of gas concentration in the oil, as was the case with the prior art devices.

This reduces the setting up time of the device 1 and also decreases the risk of underestimating the value of gas concentration in the oil, in particular when the gas-oil system is far from its thermodynamic equilibrium.

Further, if the gases reach the saturation condition inside the measuring chamber 4, they can be discharged at least partly without diminishing the reliability of the gas concentration measurements performed by the device 1.

In effect, even if transients of gas transfer through the membrane 5 are triggered by the discharge of the gases, the device 1 is able to correctly estimate the value of gas concentration in the oil by means of the non-linear function.

The method for deriving the concentration of a gas dissolved in oil preferably contemplates, when the control unit 8 detects that ($X_k$-$X_1$) is less than the predetermined value or interval $\bar{x}$ (that is, when $X_i$-$X_1$ is less than $\bar{x}$, for each

i between 2 and k) of variation of the gas concentration in the measuring chamber 4, deriving the estimated value for the gas concentration in the oil corresponding to the value $X_i$ by means of a simplified linear function for the concentration value $X_i$, that is to say, by means of a linear function of the $Y_i=f(X_i)$ type.

In effect, not exceeding the predetermined interval $\bar{x}$ of variation of the gas concentration in the measuring chamber 4 indicates a situation of small variation of the gas concentration in the oil 2 in the electrical equipment 3, which in turn indicates a condition of substantial equilibrium of the gas-oil system.

Thus, when the predetermined value or interval $\bar{x}$ of variation of the concentration is not exceeded, the device 1 uses that linear function advantageously to reduce the computational load of the control unit 8 and to simplify the calculation of the estimated value of the gas concentration in the oil.

Shown below is the linear function preferably used to calculate the estimated value $Y_i$ of gas concentration in the oil from the measured value $X_i$ of gas concentration in the measuring chamber 4.

$$Y_i = \lambda(T_g, P) * X_i$$

Where:

- $\lambda(T_g, P)$ is Ostwald's solubility coefficient.

The method for deriving the concentration of a gas dissolved in oil, illustrated schematically in Figure 2, is preferably implemented with FIFO logic (that is, the first data item in is the first data item out of the block) with reference to the block C.

In effect, the first estimated value $Y_1$ of gas concentration in the oil calculated by the control unit using the linear or non-linear function corresponds preferably to the first value $X_1$ of gas concentration acquired in the measuring chamber 4, and so on for the remaining values.

The description set out above thus defines a method for deriving the concentration of a gas dissolved in an electrical insulating oil 2 of electrical equipment, comprising the following steps:

- preparing a membrane 5 permeable to the gas, interposed between a container 7 of the oil 2 and a measuring chamber 4 that receives a part of the gas through the membrane 5;
- measuring the value of gas concentration in the measuring chamber 4;
- deriving an estimated value of the concentration of the gas in the oil 2 as a function of the measured value, characterized in that
- measuring comprises taking at successive measuring instants a plurality of measurements of the values of gas concentration in the measuring chamber 4;
- deriving comprises calculating the estimated value of gas concentration in the oil 2, at an instant selected from said measuring instants, according to a non-linear function of the values measured at the selected measuring instant and at one or more of the measuring instants preceding the one selected.

Preferably, the plurality of measurements at successive measuring instants are taken in a predetermined measuring time interval within which the measurements can be ordered sequentially from a first measurement to a last measurement.

The method preferably comprises, after at least one of the measurements taken after the first, comparing that measured value with at least one of the values preceding the plurality of measured values.

The step of deriving the estimated value of the gas concentration in the oil 2 corresponding to that measurement is performed in a mode selected according to said comparing step from the following alternatives:

- a calculation according to a non-linear function of the values measured at the selected measuring instant and at one or more of the measuring instants preceding the one selected, or
- a simplified calculation according to a linear function of the value measured at said selected measuring instant.

It should be observed that, preferably, the estimated value of gas concentration in the oil at an instant selected from said measuring instants, is calculated according to a non-linear function of the values measured at the selected measuring instant and at all the measuring instants preceding the one selected.

According to this invention, the diagnostic apparatus 11 further comprises a measurement module 10 for measuring electric pulses relating to partial electric discharges (hereinafter also referred to as PD, the abbreviation of the term Partial Discharges) which occur in the equipment 3 (more specifically, in the transformer 3), and a processing unit 12.

It should be noted that the control unit 8 and the processing unit 12 can be integrated in a single processing unit; in any case, the control unit 8 and the processing unit 12 define the processing means 9.

More specifically, the measurement module 10 for measuring electric pulses is of the electrical type the measurement module 10 is configured to measure the current pulses that travel a measuring circuit coupled with the electrical system, of the transformer 3.

The processing unit 12 is connected to the device 1 and to the measurement module 10 for measuring the partial discharges. The processing unit 12 (integrated in the control unit 8 or connected to it) is designed to derive at least one concentration parameter correlated with the gas concentration measured in a predetermined acquisition time interval and at least one discharge parameter correlated with the partial discharges measured concurrently with the same acquisition time interval).

In particular, as regards the expression "concurrently" the following should be noted.

The expression "concurrently" is used to mean that the electrical discharges the discharge parameter is correlated with might be measured in the same acquisition time interval in which the gas concentration is measured or immediately before or after that time interval, that is to say that the discharges do not necessarily have to be acquired in the same time interval in which the gas concentrations are acquired, but might also be acquired before or after that time interval, provided always that gas and PD measurement times are sufficiently close to guarantee that the measured data relating to the gas concentrations and PD signals are pertinent to the same sources.

In effect, it should be observed that, generally speaking, a defect in the insulation of the equipment 3, is at once a source of gas and a source of partial electric discharges (often, the discharges themselves, which occur in the oil or paper insulation, generate the gas).

The processing unit 12 comprises an identification module (not illustrated) connectable to a database containing reference values of predetermined indicators relating to a data set consisting at least of the concentration and discharge parameters.

These reference values of predetermined indicators contained in the database are characteristic values of predetermined categories of sources which generate the partial discharges and/or the gas dissolved in the oil.

The identification module is programmed to compare a data set composed of the values of the concentration and discharge parameters, derived by the processing unit 12, with the data in the database in order to assign that data set to one or more of those predetermined categories of sources which generate partial discharges and/or gas dissolved in the oil.

Preferably, the apparatus 11 also comprises display means (not illustrated), for example a display unit, connected to the processing unit 12 and designed to display the diagnostic indication regarding the identified sources of partial discharges and/or gas dissolved in the oil.

The operation of the diagnostic apparatus 11 is described below.

The device 1 measures the concentration of at least one gas dissolved in the insulating oil of the electrical equipment 3 (in the manner described above).

More specifically, the device 1 measures the concentrations of CO and $H_2$ in the oil in a predetermined time interval and transmits these concentrations to the processing unit 12.

The processing unit 12 derives at least one concentration parameter as a function of the measured concentration of the at least one gas dissolved in the oil.

Preferably, the processing unit 12 derives the following two concentration parameters:

- the value of CO concentration in the oil;
- and the value of $H_2$ concentration in the oil.

The measurement module 10 measures the electrical pulses relating to partial electrical discharges which occur in the oil and which generate the pulses.

More specifically, it is assumed that the transformer is subjected to alternating voltage; in light of this, it is possible to attribute to each electrical pulse (partial discharge) measured, the value of a phase parameter, given by the phase (or the value) of the voltage applied to the transformer (or to the electrical equipment 3) at the instant in which the pulse is measured.

Preferably for each pulse measured, the processing unit 12 extracts the value of parameters correlated with the waveform of the pulse.

More specifically, for each of the pulses measured, the processing unit 12 derives the following:

- the value of an amplitude parameter correlated with the amplitude of the pulse measured;
- the value of a phase parameter, representing the value of an alternating voltage applied to the electrical equipment at the instants of measuring the pulses;

- the value of a first shape parameter W correlated with the frequency content of the pulse;
- and the value of a second shape parameter T, correlated with the duration of the pulse.

It should be noted that, for deriving the above mentioned shape parameters T and W, the processing unit 12 is preferably programmed to operate as follows:

- the first shape parameter W is derived as standard deviation of the partial discharge pulse processed in the frequency domain;
- the second shape parameter T is derived as standard deviation of the partial discharge pulse processed in the time domain.

The processing unit therefore creates a data set comprising, for each of the pulses measured, the value of the aforesaid shape parameters T and W, of the amplitude parameter, correlated with the amplitude of the pulse measured, and the value of the phase parameter, representing the value of an alternating voltage applied to the electrical equipment at the instants of measuring the pulses.

Preferably, the processing unit 12 processes the data set in order to attribute the activity of partial discharges relating to that data set to one or more categories correlated with the nature of the source of the partial discharges, preferably selected from the following categories:

- internal,
- surface,
- corona.

It is specified that the expression "correlated with the nature of the source of the partial discharges" means that the categories represent the distribution of the electric field within the space region (of the defect that generates the partial discharges) where the PD occur; in effect, it should be observed that the partial discharge activity (that is, the dimension, phase and time sequence of the partial discharges that occur in sequence in a reference time interval) is closely correlated with the distribution of the electric field in the region where the discharges occur.

The "internal" category relates to an activity of partial discharges which occur in the air gaps delimited by dielectric surfaces, or dielectric solids and metal electrodes, and which have a significant component of the electric field at right angles to the surfaces (fixed gaps).

The "surface" category relates to an activity of partial discharges involving the surfaces of solid and/or liquid insulating materials and which have a significant component of the electric field tangential to the discharge surfaces.

The "corona" category relates to an activity of partial discharges which occur in air starting from a pointed element.

Preferably, the processing unit 12 compares the data of the set comprising the amplitude and phase parameters of the measured pulses with reference data contained in a database and relating to reference values adopted by the amplitude and phase parameters for the aforesaid categories of sources which generate partial discharges (that is to say, internal, surface and corona).

It should be observed that the attribution of the measured discharge activity (that is, the attribution of the measured data set) to the internal / surface / corona categories occurs by processing the data relating to the phase and amplitude of the discharges measured; preferably, this processing consists of assessing the phase-amplitude pattern associated with that data set; more specifically, the assessment is performed preferably using a fuzzy inference engine.

The data set comprising the phase and amplitude parameters of the measured pulses assigned to the aforesaid categories of partial discharge sources constitutes a discharge parameter.

Preferably, the processing unit 12 derives the following discharge parameters:

- an indication of presence or absence of partial discharges in the equipment 3 (that is, in the transformer);
- an indication of presence of intermittent partial discharges in the equipment 3 (that is, in the transformer);
- an attribution of the partial discharges measured (that is, data sets relating to a plurality of PD measured) to the internal, surface and corona categories.

Thus, the processing unit 12 defines the identification module which identifies the type of equipment insulation defect that generates the partial discharges and/or the gases dissolved in the oil.

The identification module of the processing unit 12 compares the data set composed of the values of the concentration and discharge parameters with the reference values of predetermined indicators relating to the concentration and discharge parameters contained in the database in order to attribute that data set to one or more of those predetermined categories of sources of partial discharges and/or gas.

That allows the type of source that generates the partial discharges and/or the gas dissolved in the oil to be identified from among one or more predetermined categories of partial discharges and/or gas.

More specifically, reference will be made below to the case where the equipment 3 consists of a transformer.

The diagnostic apparatus 11 is configured to identify the source (or one or more of the sources) which generate partial discharges and/or gas dissolved in the oil in a transformer from among the categories of sources listed below:

- overheating of the transformer;
- electric arcing in a core of the transformer;
- defects in paper insulation of the transformer;
- electrical discharges produced in the oil by a high voltage electrode of the transformer;
- electrical discharges in poorly impregnated zones inside the transformer;
- oil bubbles;
- discharges produced along an outside surface of the transformer insulation.

The processing unit 12 attributes the derived concentration and discharge parameter data set to the category "overheating of the transformer" if the value of CO concentration in the oil is greater than the corresponding reference value, present in the database, and in the absence of partial discharges in the transformer.

The predetermined database reference value for CO concentration takes into account the CO concentration in the oil under optimum working conditions of the transformer, that is to say, when the transformer is not overheated.

Preferably, the predetermined database reference value for CO concentration is 1500 ppm.

More preferably, the predetermined database reference value for CO concentration is 400 ppm.

The processing unit 12 attributes the derived concentration and discharge parameter data set to the category "electric arcing in a core of the transformer" if the value of $H_2$ concentration in the oil is greater than a corresponding first reference value, corresponding to a "high" concentration of $H_2$, and in the absence of partial discharges.

Preferably, the corresponding first reference value for $H_2$ concentration, corresponding to a "high" concentration of $H_2$, is 10000 ppm.

Preferably, that corresponding first reference value relates to a "high" concentration of $H_2$ in the oil.

The processing unit 12 attributes the derived concentration and discharge parameter data set to the category "electrical discharges produced in the oil by a high voltage electrode of the transformer" if the value of $H_2$ concentration in the oil is greater than the corresponding first reference value, corresponding to a "high" concentration of $H_2$, and in the presence of an activity of partial discharges attributed to the corona category.

Preferably, the corresponding first reference value for $H_2$ concentration, corresponding to a "high" concentration is 10000 ppm.

The processing unit 12 can use as further discharge parameters also the T and W shape parameters to attribute the data set to the category "electrical discharges produced in the oil by a high voltage electrode" so as to derive the diagnostic indication with a higher degree of reliability.

In effect, the aforesaid derived T and W shape parameters have, values which are, respectively, greater than a predetermined reference value (T "high") and lower than another predetermined reference value (W "low") when the source of partial discharges and/or gas are electrical discharges produced in the oil by a high-voltage electrode.

Preferably, the predetermined reference value for T is 5mS, while the predetermined reference value for W is 1Mhz.

The aforesaid reference values for T and W (5ms and 1 Mhz) apply when the signals relating to the electric pulses are carried in a passband typical of a capacitive coupler.

The processing unit 12 attributes the derived concentration and discharge parameter data set to the category "defects in paper insulation of the transformer" if the value of $H_2$ concentration in the oil is greater than a corresponding reference value, relating to a "low" concentration of $H_2$ in the oil, and in the presence of intermittent partial discharges.

Preferably, the corresponding reference value for $H_2$ concentration, corresponding to a "low" concentration of $H_2$, is 200 ppm.

The processing unit 12 attributes the derived concentration and discharge parameter data set to the category "electrical discharges in poorly impregnated zones inside the transformer" if the value of $H_2$ concentration in the oil is greater than the corresponding reference value, relating to a "high" concentration of $H_2$, and in the presence of an activity of partial discharges attributed to the internal and/or surface category.

The processing unit 12 can use as further discharge parameters also the T and W shape parameters to attribute the data set to the category "electrical discharges in poorly impregnated zones inside the transformer" so as to derive the diagnostic indication with a higher degree of reliability.

In effect, the aforesaid derived T and W shape parameters have, values which are, respectively, greater than a predetermined reference value and lower than another predetermined reference value when the source of partial discharges and/or gas are electrical discharges in poorly impregnated zones inside the transformer.

Preferably, the predetermined reference value for T is 5mS, while the predetermined reference value for W is 1Mhz.

The aforesaid reference values for T and W (5ms and 1 Mhz) apply when the signals relating to the electric pulses are carried in a passband typical of a capacitive coupler.

The processing unit 12 attributes the derived concentration and discharge parameter data set to the category "oil bubbles" in the transformer if the value of $H_2$ concentration in the oil is less than the corresponding reference value, relating to a "low" concentration of $H_2$ in the oil, and in the presence of an activity of partial discharges attributed to the internal and/or surface category.

Preferably, the reference value for $H_2$ concentration, corresponding to a "low" concentration of H2, is 200 ppm.

Further, when the processing unit 12 attributes the derived concentration and discharge parameter data set to the category "oil bubbles", the degree of belonging of the data set comprising the discharge parameters to the categories correlated with the nature of the partial discharge source (internal, surface, corona) is highest for the "internal" category.

The processing unit 12 attributes the derived concentration and discharge parameter data set to the category "electrical discharges produced along an outside surface of the transformer insulation" if the value of $H_2$ concentration in the oil is less than a first corresponding reference value, relating to a "high" concentration of $H_2$ in the oil and greater than a second corresponding reference value, relating to a "low" concentration of $H_2$ in the oil and in the presence of an activity of partial discharges attributed to the internal and/or surface category.

Preferably, the first reference value for $H_2$ concentration, corresponding to a "high" concentration of $H_2$, is 10000 ppm, and the second reference value for $H_2$ concentration, corresponding to a "low" concentration of $H_2$, is 200 ppm.

Further, when the processing unit 12 attributes the derived concentration and discharge parameter data set to the category "electrical discharges produced along an outside surface of the transformer insulation", the degree of belonging of the data set comprising the discharge parameters to the categories correlated with the nature of the partial discharge source (internal, surface, corona) is highest for the "surface" category.

Table 1 below shows the attribution of the sources of partial discharges and/or gas as a function of the values of the concentration parameter/s and of the discharge parameter/s using the diagnostic method and the diagnostic apparatus of this invention.

Table 1

| | | PDA | | | |
|---|---|---|---|---|---|
| | | absence of PD | intermittent PD | corona PD | internal- surface PD |
| DGA | concentration of H2 greater than a predetermined value (high) | electric arcing in a core | | electrical discharges from a high voltage electrode | electrical discharges in non-impregnated zones inside the transformer; |
| | concentration of H2 between two predetermined values (medium) | | | | discharges along an outside surface of the transformer. |
| | concentration of H2 less than a predetermined value (low) | | defects in paper insulation | | oil bubbles |
| | Presence of CO | overheating of the transformer; | | | |

The processing unit 12 may further comprise a filtering module, that is, a filter, configurable to select only a part of the electrical pulses relating to partial discharges measured in the acquisition time interval so as to derive the discharge parameter only on the selected part of the partial discharges.

For example, the filter allows the processing unit 12 to derive one or more discharge parameters correlated with the partial discharges and excluding electrical discharges due to predetermined types of noise, so as to advantageously derive discharge parameters which are reliable and immune to noise.

The diagnostic apparatus 11 advantageously makes it possible to obtain highly reliable diagnostic indications regarding the insulation state of electrical equipment, in particular a transformer.

In effect, the diagnostic apparatus 11 derives a diagnostic indication about the insulation conditions of electrical equipment by combining DGA with PDA.

Thus, the apparatus 11 proposed is particularly robust against uncertainty of measured data, as regards both DGA and PDA.

In effect, according to the invention, to perform a reliable diagnosis (with an excellent capacity of discernment to distinguish the type of defect) it is sufficient to measure the gases (CO e $H_2$) with the highest concentrations (and thus particularly easy and reliable to measure) and from there derive indications on the nature of the PD sources.

Compared to DGA based prior art diagnostic apparatuses, this diagnostic apparatus can provide a higher number of diagnostic indications by measuring the concentration in oil of a smaller number of gases, with obvious advantages in terms of costs and operating reliability of the diagnostic apparatus.

Furthermore, unlike the case of prior art DGA solutions, any errors in estimating the concentration of one or more gases in the oil do not significantly reduce the reliability of the diagnostic indications derived by the apparatus 11; in effect, the diagnostic information is derived using at least one concentration parameter obtained by DGA and at least one discharge parameter obtained by PDA.

Advantageously, therefore, the sensors used in the diagnostic apparatus 11 to measure the concentration of gases in the oil may be less precise and accurate than those of the prior art, DGA based apparatuses, with obvious advantages in terms of costs.

Another advantage of this invention is that it provides a diagnostic apparatus 11 that can identify in a transformer a plurality of sources which generate partial discharges and/or gas dissolved in the oil with a high degree of discernment to distinguish these sources but without complicating the apparatus.

Moreover, the diagnostic apparatus of the invention uses a fuzzy inference engine operating on the concentration and discharge parameter to derive the aforesaid diagnostic indication.

The fuzzy inference engine makes it possible to attribute the data set composed of the values of the concentration and discharge parameters to one or more categories of sources which generate partial discharges and/or gas dissolved in the oil using predetermined rules applied to the concentration and discharge parameter/parameters.

This advantageously allows even more accurate diagnostic indications to be obtained, including an indication about the certainty (or uncertainty) of the indication provided, usually referred to by the term "likelihood".

In other embodiments of the diagnostic apparatus, other diagnostic indications about the insulation conditions of a transformer are derived on the basis of a data set consisting of a combination of one or more concentration parameters and one or more discharge parameters from among those set out above.

In any event, the data set comprising at least one concentration parameter and one discharge parameter must include a combination of concentration and discharge parameters from which at least one of the above mentioned sources of electrical discharges and/or gas must be identifiable.

Moreover, the apparatus 11 can derive further concentration and discharge parameters to improve the reliability of the diagnostic indications derived therefrom compared to those described above.

The description set out above also defines a diagnostic method for assessing the insulation condition of electrical equipment 3 insulated with oil 2, comprising the following steps:

- measuring the concentration of at least one gas dissolved in the insulating oil 2 in the electrical equipment 3;
- deriving at least one concentration parameter correlated with the gas concentration measured in a predetermined acquisition time interval,
- measuring electrical pulses relating to partial electrical discharges which occur in the electrical equipment 3 and which generate said pulses;
- deriving at least one discharge parameter correlated with the partial discharges measured substantially concurrently with the predetermined acquisition time interval;
- deriving a diagnostic indication about the insulation condition of the electrical equipment 3 as a function of the derived values of the concentration parameter and of the discharge parameter, in combination.

Preferably, in this method, the step of deriving the diagnostic indication comprises the following steps:

- preparing a database containing reference values of predetermined indicators relating to a data set comprising at least said concentration and discharge parameters, said reference values being characteristic of predetermined categories of sources that generate the partial discharges and/or the gas dissolved in the oil;
- comparing a data set composed of derived values of the concentration and discharge parameters with the data in the database in order to assign said data set to one or more of said source categories, thereby identifying the type of source that generates the partial discharges and/or the gas dissolved in the oil.

In another embodiment of the diagnostic method, the step of comparing a data set composed of derived values of the concentration and discharge parameters with the data in the database in order to assign said data set to one or more of said source categories is performed in order to provide a signal regarding the insulation condition of the electrical equipment 3.

The signal may comprise information regarding the state of the insulation (for example, a traffic light which is green if the state of the insulation is good or red if the insulation is not in good condition and the electrical equipment requires attention) or it may comprise information regarding the operation to be carried out on the transformer.

Further, in yet another embodiment of the diagnostic method, the step of deriving a diagnostic indication comprises using a fuzzy inference engine operating on the at least one concentration parameter and on the at least one discharge parameter in order to derive said diagnostic indication.

It will be understood that the invention described is susceptible of industrial application and may be modified and adapted in several ways without thereby departing from the scope of the appended claims.

**Claims**

1. A diagnostic method for assessing the insulation condition of electrical equipment (3) insulated with oil (2), comprising the following steps:

   - measuring the concentration of at least one gas dissolved in the insulating oil (2) in the electrical equipment (3);
   - deriving at least one concentration parameter correlated with the gas concentration measured in a predetermined acquisition time interval,
   - measuring electrical pulses relating to partial electrical discharges which occur in the electrical equipment (3) and which generate said pulses;
   - deriving at least one discharge parameter correlated with the partial discharges measured substantially concurrently with the predetermined acquisition time interval;
   - deriving a diagnostic indication about the insulation condition of the electrical equipment (3) as a function of the derived values of the concentration parameter and of said at least one discharge parameter, in combination, **characterized in that** the following discharge parameters are derived:
   - an indication of presence or absence of partial discharges in the equipment (3);
   - an indication of presence of intermittent partial discharges in the equipment (3); and
   - an attribution of the measured partial discharges to internal, surface and corona categories,

   wherein the attribution of the measured discharge activity to the internal, surface and corona categories occurs by processing data relating to a phase parameter and an amplitude parameter of the measured discharges.

2. The method according to claim 1, wherein the step of deriving the diagnostic indication comprises the following steps:

   - preparing a database containing reference values of predetermined indicators relating to a data set comprising at least said concentration and discharge parameters, said reference values being characteristic of predetermined categories of sources that generate the partial discharges and/or the gas dissolved in the oil;
   - comparing a data set composed of derived values of the concentration and discharge parameters with the data in the database in order to assign said data set to one or more of said source categories in order to provide a signal regarding the insulation condition of the electrical equipment (3).

3. The method according to claim 1, wherein the step of deriving the diagnostic indication comprises the following steps:

   - preparing a database containing reference values of predetermined indicators relating to a data set comprising at least said concentration and discharge parameters, said reference values being characteristic of predetermined categories of sources that generate the partial discharges and/or the gas dissolved in the oil;
   - comparing a data set composed of derived values of the concentration and discharge parameters with the data in the database in order to assign said data set to one or more of said source categories, thereby identifying the type of source that generates the partial discharges and/or the gas dissolved in the oil.

4. The method according to claim 3, wherein the electrical equipment is a transformer and the predetermined categories of sources that generate the partial discharges and/or the gas dissolved in the oil comprise one or more of the categories from the following list:

   - overheating of the transformer;

- electric arcing in a core of the transformer;
- defects in paper insulation of the transformer;
- electrical discharges produced in the oil by a high voltage electrode of the transformer;
- electrical discharges in poorly impregnated zones inside the transformer;
- oil bubbles;
- discharges produced along an outside surface of the transformer insulation.

**5.** The method according to claim 4, comprising deriving a concentration of CO in the oil, constituting said at least one concentration parameter, and an indication of the presence or absence of partial discharges measured in the transformer, constituting said at least one discharge parameter, the measured data set being assigned to the category of overheating of the transformer if the value of CO concentration in the oil is greater than a corresponding reference value and in the absence of partial discharges.

**6.** The method according to claim 4 or 5, comprising deriving a concentration of $H_2$ in the oil (2), constituting said at least one concentration parameter, and an indication of the presence or absence of partial discharges measured in the transformer, constituting said at least one discharge parameter, the measured data set being assigned to the category of electric arcing in a core of the transformer if the value of $H_2$ concentration in the oil is greater than a corresponding reference value and in the absence of partial discharges.

**7.** The method according to any of the claims from 4 to 6, comprising deriving a concentration of $H_2$ in the oil (2), constituting said at least one concentration parameter, and an indication of the presence or absence of partial intermittent discharges measured in the transformer, constituting said at least one discharge parameter, the measured data set being assigned to the category of defects in paper insulation of the transformer if the value of $H_2$ concentration in the oil is greater than a corresponding reference value and in the presence of partial intermittent discharges.

**8.** The method according to any of the claims from 4 to 7, wherein the step of deriving the discharge parameters comprises:

- generating a data set comprising, for each of the pulses measured, the value of an amplitude parameter, correlated with the amplitude of the pulse measured, and the value of a phase parameter, representing the value of an alternating voltage applied to the electrical equipment at the instants of measuring the pulses, and
- processing the data set in order to assign the activity of partial discharges relating to said data set to one or more categories correlated with the nature of the source of the partial discharges, selected from the following categories: internal, surface and corona,

the assigned categories of partial discharge sources constituting the at least one discharge parameter.

**9.** The method according to claim 8, comprising deriving a concentration of $H_2$ in the oil (2), constituting said at least one concentration parameter, the measured data set being assigned to the category of electrical discharges produced in the oil by a high voltage electrode of the transformer if the value of $H_2$ concentration in the oil is greater than a corresponding reference value and in the presence of an activity of partial discharges assigned to the corona category.

**10.** The method according to claim 8 or 9, comprising deriving a concentration of $H_2$ in the oil (2), constituting said at least one concentration parameter, the measured data set being assigned to the category of electrical discharges in poorly impregnated zones inside the transformer if the value of $H_2$ concentration in the oil is greater than a corresponding reference value and in the presence of an activity of partial discharges assigned to the surface or corona category.

**11.** The method according to any of the claims from 8 to 10, comprising deriving a concentration of $H_2$ in the oil (2), constituting said at least one concentration parameter, the measured data set being assigned to the category of oil bubbles in the transformer if the value of $H_2$ concentration in the oil is less than a corresponding reference value and in the presence of an activity of partial discharges assigned to the surface or corona category.

**12.** The method according to any of the claims from 8 to 11, comprising deriving a concentration of $H_2$ in the oil (2), constituting said at least one concentration parameter, the measured data set being assigned to the category of discharges produced along an outside surface of the transformer insulation if the value of $H_2$ concentration in the oil is less than a corresponding first reference value and greater than a corresponding second reference value less than the first reference value, and in the presence of an activity of partial discharges assigned to the surface or corona category.

13. The method according to any of the foregoing claims, wherein the step of deriving a diagnostic indication comprises using a fuzzy inference engine operating on the at least one concentration parameter and on the at least discharge parameter in order to derive said diagnostic indication.

14. The method according to any of the foregoing claims, wherein the step of measuring the concentration of a gas dissolved in the insulating oil of the electrical equipment comprises the steps of:

- preparing a membrane (5) permeable to the gas, interposed between a container (7) of the oil (2) and a measuring chamber (4) that receives a part of the gas through the membrane (5);
- taking at successive measuring instants a plurality of measurements of the values of gas concentration in the measuring chamber (4) which is separated from the oil container by the permeable membrane (5);
- deriving a value of gas concentration in the oil (2) at an instant selected from said measuring instants, according to a non-linear function of the values measured at the selected measuring instant and at one or more of the measuring instants preceding the one selected.

15. A diagnostic apparatus (11) for assessing the insulation condition of electrical equipment (3) insulated with oil (2), equipped with a device (1) for measuring at least the concentration of a gas dissolved in the insulating oil (2) of the electrical equipment (3), comprising, combined together:

- a module (10) for measuring electrical pulses relating to partial electrical discharges which occur in the electrical equipment (3) and which generate said pulses;
- a processing unit (12) connected to the device (1) and to the module (10) for measuring the partial discharges and designed to derive at least one concentration parameter correlated with the gas concentration and at least one discharge parameter correlated with the partial discharges and to derive a diagnostic indication about the insulation condition of the electrical equipment (3), as a function of the derived values of the at least one concentration parameter and the at least one discharge parameter, in combination,

characterized in that the processing unit (12) is configured for deriving the following discharge parameters:

- an indication of presence or absence of partial discharges in the equipement (3);
- an indication of presence of intermittent partial discharges in the equipment (3); and
- an attribution of the measured partial discharges to internal, surface and corona categories,

wherein the processing unit (12) is configured for processing data relating to a phase parameter and an amplitude parameter of the measured discharges, to provide said attribution of the measured discharge activity to the internal, surface and corona categories.

16. The apparatus according to claim 15, comprising an identification module which can be connected to a data base containing reference values of predetermined indicators relating to a data set comprising at least said concentration and discharge parameters, said reference values being characteristic of predetermined source categories that generate partial discharges and/or the gas dissolved in the oil, said identification module being programmed to compare a data set composed of derived values of the concentration and discharge parameters with the data in the database in order to assign said data set to one or more of said source categories, thereby identifying the type of source that generates the partial discharges and/or the gas dissolved in the oil.

17. The apparatus according to claim 16, wherein the electrical equipment is a transformer and the identification module is adapted to identify one or more of the categories from the following list of the said predetermined categories of sources that generate the partial discharges and/or the gas dissolved in the oil:

- overheating of the transformer;
- electric arcing in a core of the transformer;
- defects in paper insulation of the transformer;
- electrical discharges produced in the oil by a high voltage electrode of the transformer;
- electrical discharges in poorly impregnated zones inside the transformer;

- oil bubbles;
- discharges produced along an outside surface of the transformer insulation.

18. The apparatus according to any of the claims from 15 to 17, wherein the device (1) comprises:

- a membrane (5) permeable to the gas, interposed between a container (7) of the oil (2) and a measuring chamber (4) that receives a part of the gas through the membrane (5);
- a sensor (6) mounted in the measuring chamber (4) to measure a value of the gas concentration in the measuring chamber (4);
- a control unit (8) connected to the sensor (6) to derive an estimated value of gas concentration in the oil according to the value measured in the measuring chamber (4),

**characterized in that** the control unit (8) is designed to take, at successive measuring instants, a plurality of measurements of the values of gas concentration in the measuring chamber (4) and to calculate the estimated value of gas concentration in the oil at an instant selected from said measuring instants, according to a non-linear function of the values measured at the selected measuring instant and at one or more of the measuring instants preceding the one selected.

**Patentansprüche**

1. Diagnoseverfahren zum Überprüfen des Isolationszustandes eines mit Öl (2) isolierten elektrischen Gerätes (3), umfassend die folgenden Schritte:

- Messen der Konzentration mindestens eines Gases, das im Isolieröl (2) im elektrischen Gerät (3) gelöst ist;
- Bestimmen mindestens eines Konzentrationsparameters, der mit der Gaskonzentration korreliert ist, die in einem festgelegten Erfassungszeitintervall gemessen wird;
- Messen von elektrischen Impulsen, die sich auf elektrische Teilentladungen beziehen, die im elektrischen Gerät (3) auftreten und die Impulse erzeugen;
- Bestimmen mindestens eines Entladungsparameters, der mit den Teilentladungen korreliert ist, die im Wesentlichen gleichzeitig mit dem festgelegten Erfassungszeitintervall gemessen werden;
- Bestimmen einer Diagnoseanzeige zum Isolationszustand des elektrischen Gerätes (3) gemäß den bestimmten Werten des Konzentrationsparameters und des mindestens einen Entladungsparameters in Kombination,

**dadurch gekennzeichnet, dass** die folgenden Entladungsparameter bestimmt werden:

- eine Anzeige des Vorhandenseins oder Nichtvorhandenseins von Teilentladungen im Gerät (3);
- eine Anzeige des Vorhandenseins von intermittierenden Teilentladungen im Gerät (3); und
- eine Zuteilung der gemessenen Teilentladungen zu der Innen-, Oberfläche- und Korona-Kategorie,

wobei die Zuteilung der gemessenen Entladungsaktivität zu der Innen-, Oberfläche und Korona-Kategorie durch die Verarbeitung von Daten auftritt, die sich auf einen Phasenparameter und einen Amplitudenparameter der gemessenen Entladungen beziehen.

2. Verfahren nach Anspruch 1, wobei der Schritt des Bestimmens der Diagnoseanzeige die folgenden Schritte umfasst:

- Vorbereiten einer Datenbank, enthaltend Referenzwerte festgelegter Anzeiger, die sich auf einen Datensatz beziehen, umfassend mindestens die Konzentrations- und Entladungsparameter, wobei die Referenzwerte kennzeichnend für festgelegte Kategorien von Quellen sind, welche die Teilentladungen und/oder das im Öl gelöste Gas erzeugen;
- Vergleichen eines Datensatzes, der sich aus bestimmten Werten der Konzentrations- und Entladungsparameter zusammensetzt, mit den Daten in der Datenbank, um den Datensatz einer oder mehreren der Quellenkategorien zuzuordnen, um ein Signal bereitzustellen, das den Isolierungszustand des elektrischen Gerätes (3) betrifft.

3. Verfahren nach Anspruch 1, wobei der Schritt des Bestimmens der Diagnoseanzeige die folgenden Schritte umfasst:

- Vorbereiten einer Datenbank, enthaltend Referenzwerte festgelegter Anzeiger, die sich auf einen Datensatz beziehen, umfassend mindestens die Konzentrations- und Entladungsparameter, wobei die Referenzwerte kennzeichnend für festgelegte Kategorien von Quellen sind, welche die Teilentladungen und/oder das im Öl gelöste Gas erzeugen;

- Vergleichen eines Datensatzes, der sich aus bestimmten Werten der Konzentrations- und Entladungsparameter zusammensetzt, mit den Daten in der Datenbank, um den Datensatz einem oder mehreren der Quellenkategorien zuzuordnen, wobei die Art von Quelle identifiziert wird, welche die Teilentladungen und/oder das im Öl gelöste Gas erzeugt.

4. Verfahren nach Anspruch 3, wobei das elektrische Gerät ein Transformator ist und die festgelegten Kategorien von Quellen, welche die Teilentladungen und/oder das im Öl gelöste Gas erzeugen, eine oder mehrere Kategorien aus der folgenden Liste umfassen:

- Überhitzen des Transformators;
- elektrische Lichtbogenbildung in einem Kern des Transformators;
- Defekte in der Papierisolierung des Transformators;
- elektrische Entladungen, die im Öl durch eine Hochspannungselektrode des Transformators produziert werden;
- elektrische Entladungen in schlecht imprägnierten Bereichen innerhalb des Transformators;
- Ölblasen;
- Entladungen, die entlang einer Außenfläche der Transformatorisolierung produziert werden.

5. Verfahren nach Anspruch 4, umfassend das Bestimmen einer Konzentration von CO im Öl, bildend den mindestens einen Konzentrationsparameter, und eine Anzeige des Vorhandenseins oder Nichtvorhandenseins von im Transformator gemessenen Teilentladungen, bildend den mindestens einen Entladungsparameter, wobei der gemessene Datensatz in dem Fall, dass der Wert der CO-Konzentration im Öl größer als ein entsprechender Referenzwert ist, und im Falle des Nichtvorhandenseins von Teilentladungen, der Kategorie des Überhitzens des Transformators zugeordnet wird.

6. Verfahren nach Anspruch 4 oder 5, umfassend das Bestimmen einer Konzentration von $H_2$ im Öl (2), bildend den mindestens einen Konzentrationsparameter, und eine Anzeige des Vorhandenseins oder Nichtvorhandenseins von im Transformator gemessenen Teilentladungen, bildend den mindestens einen Entladungsparameter, wobei der gemessene Datensatz in dem Fall, dass der Wert der $H_2$-Konzentration im Öl größer als ein entsprechender Referenzwert ist, und im Falle des Nichtvorhandenseins von Teilentladungen, der Kategorie der elektrischen Lichtbogenbildung in einem Kern des Transformators zugeordnet wird.

7. Verfahren nach einem der Ansprüche 4 bis 6, umfassend das Bestimmen einer Konzentration von $H_2$ im Öl (2), bildend den mindestens einen Konzentrationsparameter, und eine Anzeige des Vorhandenseins oder Nichtvorhandenseins von im Transformator gemessenen intermittierenden Teilentladungen, bildend den mindestens einen Entladungsparameter, wobei der gemessene Datensatz in dem Fall, dass der Wert der $H_2$-Konzentration im Öl größer als ein entsprechender Referenzwert ist, und im Falle des Vorhandenseins von intermittierenden Teilentladungen, der Kategorie von Defekten in der Papierisolierung des Transformators zugeordnet wird.

8. Verfahren nach einem der Ansprüche 4 bis 7, wobei der Schritt des Bestimmens der Entladungsparameter Folgendes umfasst:

- Erzeugen eines Datensatzes, umfassend, für jeden der gemessenen Impulse, den Wert eines Amplitudenparameters, der mit der Amplitude des gemessenen Impulses korreliert ist, und den Wert eines Phasenparameters, der den Wert einer Wechselspannung, die am elektrischen Gerät in den Momenten des Messens der Impulse angelegt wird, repräsentiert, und
- Verarbeiten des Datensatzes, um die Aktivität von Teilentladungen, welche sich auf den Datensatz beziehen, einer oder mehreren Kategorien zuzuordnen, die mit der Natur der Quelle der Teilentladungen korreliert sind, die aus einer der folgenden Kategorien ausgewählt werden: Innen-, Oberfläche- und Korona-Kategorie,

wobei die zugeordneten Kategorien von Quellen der Teilentladungen den mindestens einen Entladungsparameter bilden.

9. Verfahren nach Anspruch 8, umfassend das Bestimmen einer Konzentration von $H_2$ im Öl (2), bildend den mindestens einen Konzentrationsparameter, wobei der gemessene Datensatz in dem Fall, dass der Wert der $H_2$-Konzentration im Öl größer als ein entsprechender Referenzwert ist, und im Falle des Vorhandenseins einer Aktivität von der Korona-Kategorie zugeordneten Teilentladungen, der Kategorie der im Öl durch eine Hochspannungselektrode des Transformators produzierten elektrischen Entladungen zugeordnet wird.

10. Verfahren nach Anspruch 8 oder 9, umfassend das Bestimmen einer Konzentration von $H_2$ im Öl (2), bildend den mindestens einen Konzentrationsparameter, wobei der gemessene Datensatz in dem Fall, dass der Wert der $H_2$-Konzentration im Öl größer als ein entsprechender Referenzwert ist, und im Falle des Vorhandenseins einer Aktivität von der Oberfläche- oder Korona-Kategorie zugeordneten Teilentladungen, der Kategorie elektrischer Entladungen in schlecht imprägnierten Bereichen innerhalb des Transformators zugeordnet wird.

11. Verfahren nach einem der Ansprüche 8 bis 10, umfassend das Bestimmen einer Konzentration von $H_2$ im Öl (2), bildend den mindestens einen Konzentrationsparameter, wobei der gemessene Datensatz in dem Fall, dass der Wert der $H_2$-Konzentration im Öl kleiner als ein entsprechender Referenzwert ist, und im Falle des Vorhandenseins einer Aktivität von der Oberfläche- oder Korona-Kategorie zugeordneten Teilentladungen, der Kategorie Ölblasen im Transformator zugeordnet wird.

12. Verfahren nach einem der Ansprüche 8 bis 11, umfassend das Bestimmen einer Konzentration von $H_2$ im Öl (2), bildend den mindestens einen Konzentrationsparameter, wobei der gemessene Datensatz in dem Fall, dass der Wert der $H_2$-Konzentration im Öl kleiner als ein entsprechender erster Referenzwert ist und größer als ein entsprechender zweiter Referenzwert ist, der kleiner als der erste Referenzwert ist, und im Falle des Vorhandenseins einer Aktivität von der Oberfläche- oder Korona-Kategorie zugeordneten Teilentladungen, der Kategorie von entlang einer Außenfläche der Transformatorisolierung produzierten Entladungen zugeordnet wird.

13. Verfahren nach einem der vorangehenden Ansprüche, wobei der Schritt des Bestimmens einer Diagnoseanzeige die Verwendung einer Fuzzy-Inferenzmaschine umfasst, die mit dem mindestens einen Konzentrationsparameter und dem mindestens einen Entladungsparameter arbeitet, um die Diagnoseanzeige zu bestimmen.

14. Verfahren nach einem der vorangehenden Ansprüche, wobei der Schritt des Messens der Konzentration eines Gases, das im Isolieröl des elektrischen Gerätes gelöst ist, die folgenden Schritte umfasst:

    - Vorbereiten einer für das Gas durchlässigen Membran (5), die zwischen einem Behälter (7) des Öls (2) und einer Messkammer (4) liegt, welche einen Teil des Gases durch die Membran (5) empfängt;
    - Vornehmen einer Vielzahl von Messungen der Werte der Gaskonzentration in der Messkammer (4), die vom Ölbehälter durch die durchlässige Membran (5) getrennt ist, in aufeinanderfolgenden Messmomenten;
    - Bestimmen eines Wertes der Gaskonzentration im Öl (2) in einem aus den Messmomenten ausgewählten Moment gemäß einer nicht-linearen Funktion der Werte, die im ausgewählten Messmoment und in einem oder mehreren Messmomenten gemessen wird, die dem ausgewählten vorausgehen.

15. Diagnosevorrichtung (11) zum Überprüfen des Isolationszustandes eines mit Öl (2) isolierten elektrischen Gerätes (3), das mit einem Gerät (1) zum Messen zumindest der Konzentration von Gas, das im Isolieröl (2) des elektrischen Gerätes (3) gelöst ist, ausgestattet ist, in Kombination umfassend:

    - ein Modul (10) zum Messen von elektrischen Impulsen, die sich auf elektrische Teilentladungen beziehen, die im elektrischen Gerät (3) auftreten und die Impulse erzeugen;
    - eine Verarbeitungseinheit (12), die mit der Einrichtung (1) und dem Modul (10) zum Messen der Teilentladungen verbunden und dazu konzipiert ist, mindestens einen Konzentrationsparameter, der mit der Gaskonzentration korreliert ist, und mindestens einen Entladungsparameter, der mit den Teilentladungen korreliert ist, zu bestimmen, und eine Diagnoseanzeige zum Isolationszustand des elektrischen Gerätes (3) gemäß den bestimmten Werten des mindestens einen Konzentrationsparameters und des mindestens einen Entladungsparameters in Kombination zu bestimmen, **dadurch gekennzeichnet, dass** die Verarbeitungseinheit (12) zum Bestimmen der folgenden Entladungsparameter konfiguriert ist:
    - eine Anzeige des Vorhandenseins oder Nichtvorhandenseins von Teilentladungen im Gerät (3);
    - eine Anzeige des Vorhandenseins von intermittierenden Teilentladungen im Gerät (3); und
    - eine Zuteilung der gemessenen Teilentladungen zu der Innen-, Oberfläche- und Korona-Kategorie,

wobei die Verarbeitungseinheit (12) dazu konfiguriert ist, Daten, die sich auf einen Phasenparameter und einen Amplitudenparameter der gemessenen Entladungen beziehen, zu verarbeiten, um die Zuteilung der gemessenen Entladungsaktivität zu der Innen-, Oberfläche- und Korona-Kategorie bereitzustellen.

**16.** Vorrichtung nach Anspruch 15, umfassend ein Identifizierungsmodul, das mit einer Datenbank verbunden sein kann, welche die Referenzwerte von festgelegten Indikatoren enthält, die sich auf einen Datensatz beziehen, der mindestens die Konzentrations- und Entladungsparameter umfasst, wobei die Referenzwerte kennzeichnend für festgelegte Kategorien von Quellen sind, welche die Teilentladungen und/oder das im Öl gelöste Gas erzeugen, wobei das Identifizierungsmodul dazu programmiert ist, einen Datensatz, der sich aus bestimmten Werten der Konzentrations- und Entladungsparameter zusammensetzt, mit den Daten in der Datenbank zu vergleichen, um den Datensatz einer oder mehreren der Quellenkategorien zuzuordnen, wobei die Art von Quelle identifiziert wird, welche die Teilentladungen und/oder das im Öl gelöste Gas erzeugt.

**17.** Vorrichtung nach Anspruch 16, wobei das elektrische Gerät ein Transformator ist, und das Identifizierungsmodul dazu geeignet ist, eine oder mehrere der Kategorien aus der folgenden Liste der festgelegten Kategorien von Quellen zu identifizieren, welche die Teilentladungen und/oder das im Öl gelöste Gas erzeugen:

- Überhitzen des Transformators;
- elektrische Lichtbogenbildung in einem Kern des Transformators;
- Defekte in der Papierisolierung des Transformators;
- elektrische Entladungen, die im Öl durch eine Hochspannungselektrode des Transformators produziert werden;
- elektrische Entladungen in schlecht imprägnierten Bereichen innerhalb des Transformators;
- Ölblasen;
- Entladungen, die entlang einer Außenfläche der Transformatorisolierung produziert werden.

**18.** Vorrichtung nach einem der Ansprüche 15 bis 17, wobei die Einrichtung (1) Folgendes umfasst:

- eine für das Gas durchlässige Membran (5), die zwischen einem Behälter (7) des Öls (2) und einer Messkammer (4) liegt, welche einen Teil des Gases durch die Membran (5) empfängt;
- einen Sensor (6), der in der Messkammer (4) angebracht ist, um einen Wert der Gaskonzentration in der Messkammer (4) zu messen;
- eine Steuereinheit (8), die mit dem Sensor (6) verbunden ist, um einen Schätzwert der Gaskonzentration im Öl gemäß dem Wert zu bestimmen, der in der Messkammer (4) gemessen wird,

**dadurch gekennzeichnet, dass** die Steuereinheit (8) dazu konzipiert ist, in aufeinanderfolgenden Messmomenten eine Vielzahl von Messungen der Werte der Gaskonzentration in der Messkammer (4) vorzunehmen, und den Schätzwert der Gaskonzentration im Öl in einem aus den Messmomenten ausgewählten Moment gemäß einer nicht-linearen Funktion der Werte zu berechnen, die im ausgewählten Messmoment und in einem oder mehreren Messmomenten gemessen werden, die dem ausgewählten vorausgehen.

## Revendications

**1.** Procédé de diagnostic pour l'évaluation de l'état d'un équipement électrique (3), isolé par de l'huile (2), comprenant les étapes suivantes :

- mesurer la concentration d'au moins un gaz dissous dans l'huile isolante (2) dans l'équipement électrique (3) ;
- déduire au moins un paramètre de concentration corrélé à la concentration de gaz mesurée dans un intervalle de temps d'acquisition prédéterminé,
- mesurer les impulsions électriques relatives aux décharges électriques partielles se produisant dans l'équipement électrique (3) et qui génèrent lesdites impulsions ;
- déduire au moins un paramètre de décharge corrélé aux décharges partielles mesurées essentiellement simultanément à l'intervalle de temps d'acquisition prédéterminé ;
- déduire une indication de diagnostic au sujet de l'état d'isolation de l'équipement électrique (3) en fonction des valeurs dérivées du paramètre de concentration et dudit au moins un paramètre de décharge combinés,

**caractérisé en ce que** les paramètres de décharge suivants sont déduits :

- une indication de la présence ou de l'absence de décharges partielles dans l'équipement (3) ;
- une indication de la présence de décharges partielles intermittentes dans l'équipement (3) ; et
- une attribution des décharges partielles mesurées aux catégories interne, surface et couronne,

dans lequel l'attribution de l'activité de décharge mesurée aux catégories interne, surface et couronne a lieu par le traitement de données relatives à un paramètre de phase et un paramètre d'amplitude des décharges mesurées.

2. Procédé selon la revendication 1, dans lequel l'étape de déduction de l'indication de diagnostic comprend les étapes suivantes :

- préparer une base de données contenant les valeurs de référence des indicateurs prédéterminés relatifs à un ensemble de données comprenant au moins lesdits paramètres de concentration et de décharge, lesdites valeurs de référence étant caractéristiques de catégories prédéterminées de sources générant les décharges partielles et/ou le gaz dissous dans l'huile ;
- comparer un ensemble de données composé des valeurs dérivées des paramètres de concentration et de décharge avec les données contenues dans la base de données afin d'affecter ledit ensemble de données à une ou plusieurs desdites catégories de source afin de fournir un signal concernant l'état d'isolation de l'équipement électrique (3).

3. Procédé selon la revendication 1, dans lequel l'étape de déduction de l'indication de diagnostic comprend les étapes suivantes :

- préparer une base de données contenant les valeurs de référence d'indicateurs prédéterminés relatifs à un ensemble de données comprenant au moins lesdits paramètres de concentration et de décharge, lesdites valeurs de référence étant caractéristiques de catégories prédéterminées de sources générant les décharges partielles et/ou le gaz dissous dans l'huile ;
- comparer un ensemble de données composé des valeurs dérivées des paramètres de concentration et de décharge avec les données contenues dans la base de données afin d'affecter ledit ensemble de données à une ou plusieurs desdites catégories de source, identifiant par conséquent le type de source qui génère les décharges partielles et/ou le gaz dissous dans l'huile.

4. Procédé selon la revendication 3, dans lequel l'équipement électrique est un transformateur et les catégories prédéterminées de sources générant les décharges partielles et/ou le gaz dissous dans l'huile comprennent une ou plusieurs des catégories contenues dans la liste ci-après :

- surchauffe du transformateur ;
- production d'arcs au coeur du transformateur ;
- défauts de l'isolation en papier du transformateur ;
- décharges électriques produites dans l'huile par une électrode à haute tension du transformateur ;
- décharges électriques dans des zones faiblement imprégnées à l'intérieur du transformateur ;
- bulles dans l'huile ;
- décharges produites le long d'une surface extérieure de l'isolation du transformateur.

5. Procédé selon la revendication 4, comprenant la déduction d'une concentration de CO dans l'huile, constituant ledit au moins un paramètre de concentration et une indication de la présence ou de l'absence de décharges partielles mesurées dans le transformateur, constituant ledit au moins un paramètre de décharge, l'ensemble des données mesurées étant affecté à la catégorie désignée par la surchauffe du transformateur si la valeur de la concentration de CO dans l'huile est supérieure à une valeur de référence correspondante et en l'absence de décharges partielles.

6. Procédé selon les revendications 4 ou 5, comprenant la déduction d'une concentration de $H_2$ dans l'huile (2), constituant ledit au moins un paramètre de concentration et une indication de la présence ou de l'absence de décharges partielles mesurées dans le transformateur, constituant ledit au moins un paramètre de décharge, l'ensemble des données mesurées étant affecté à la catégorie désignée par la production d'arcs au coeur du transformateur si la valeur de la concentration de $H_2$ dans l'huile est supérieure à une valeur de référence correspondante et en l'absence de décharges partielles.

7. Procédé selon l'une quelconque des revendications de 4 à 6, comprenant la déduction d'une concentration de $H_2$ dans l'huile (2), constituant ledit au moins un paramètre de concentration et une indication de la présence ou de l'absence de décharges intermittentes partielles mesurées dans le transformateur, constituant ledit au moins un paramètre de décharge, l'ensemble des données mesurées étant affecté à la catégorie désignée par les défauts de l'isolation en papier du transformateur si la valeur de la concentration de $H_2$ dans l'huile est supérieure à une valeur de référence correspondante et en la présence de décharges intermittentes partielles.

**8.** Procédé selon l'une quelconque des revendications de 4 à 7, dans lequel l'étape de déduction des paramètres de décharge comprend :

- générer un ensemble de données comprenant, pour chacune des impulsions mesurées, la valeur d'un paramètre d'amplitude, corrélé à l'amplitude de l'impulsion mesurée et la valeur d'un paramètre de phase, représentant la valeur d'une tension alternative appliquée à l'équipement électrique aux instants de mesure des impulsions, et
- traiter l'ensemble des données afin d'affecter l'activité des décharges partielles relatives au dit ensemble de données à une ou plusieurs catégories corrélées à la nature de la source des décharges partielles, sélectionnée parmi les catégories suivantes : interne, surface et couronne,

les catégories affectées des sources de décharge partielle constituant l'au moins un paramètre de décharge.

**9.** Procédé selon la revendication 8, comprenant la déduction d'une concentration de $H_2$ dans l'huile (2), constituant ledit au moins un paramètre de concentration, l'ensemble des données mesurées étant affecté à la catégorie désignée par les décharges électriques produites dans l'huile par une électrode à haute tension du transformateur si la valeur de la concentration de $H_2$ dans l'huile est supérieure à une valeur de référence correspondante et en la présence d'une activité de décharges partielles affectée à la catégorie couronne.

**10.** Procédé selon les revendications 8 ou 9, comprenant la déduction d'une concentration de $H_2$ dans l'huile (2), constituant ledit au moins un paramètre de concentration, l'ensemble des données mesurées étant affecté à la catégorie désignée par les décharges électriques dans des zones faiblement imprégnées à l'intérieur du transformateur si la valeur de la concentration de $H_2$ dans l'huile est supérieure à une valeur de référence correspondante et en la présence d'une activité de décharges partielles affectée à la catégorie surface ou couronne.

**11.** Procédé selon l'une quelconque des revendications de 8 à 10, comprenant la déduction d'une concentration de $H_2$ dans l'huile (2), constituant ledit au moins un paramètre de concentration, l'ensemble des données mesurées étant affecté à la catégorie désignée par des bulles dans l'huile dans le transformateur si la valeur de la concentration de $H_2$ dans l'huile est inférieure à une valeur de référence correspondante et en la présence d'une activité de décharges partielles affectée à la catégorie surface ou couronne.

**12.** Procédé selon l'une quelconque des revendications de 8 à 11, comprenant la déduction d'une concentration de $H_2$ dans l'huile (2), constituant ledit au moins un paramètre de concentration, l'ensemble des données mesurées étant affecté à la catégorie désignée par les décharges produites le long d'une surface extérieure de l'isolation du transformateur si la valeur de la concentration de $H_2$ dans l'huile est inférieure à une valeur de référence correspondante et supérieure à une seconde valeur de référence correspondante inférieure à la première valeur de référence et en la présence d'une activité de décharges partielles affectée à la catégorie surface ou couronne.

**13.** Procédé selon l'une quelconque des revendications précédentes, dans lequel l'étape de déduction d'une indication de diagnostic comprend l'utilisation d'un moteur d'inférence floue agissant sur l'au moins un paramètre de concentration et sur l'au moins un paramètre de décharge afin de déduire ladite indication de diagnostic.

**14.** Procédé selon l'une quelconque des revendications précédentes, dans lequel l'étape consistant à mesurer la concentration d'un gaz dissous dans l'huile isolante de l'équipement électrique comprend les étapes suivantes :

- préparer une membrane (5) perméable au gaz, interposée entre un récipient (7) d'huile (2) et une chambre de mesure (4) qui reçoit une partie du gaz à travers la membrane (5) ;
- relever, à des instants de mesure successifs, une pluralité de mesures des valeurs de concentration de gaz dans la chambre de mesure (4) étant séparée du récipient d'huile par la membrane perméable (5) ;
- déduire une valeur de la concentration de gaz dans l'huile (2) à un instant sélectionné à partir desdits instants de mesure, selon une fonction non-linéaire des valeurs mesurées à l'instant de mesure sélectionné et à un ou plusieurs des instants de mesure précédents celui sélectionné.

**15.** Appareil de diagnostic (11) permettant d'évaluer l'état d'isolation d'un équipement électrique (3) isolé par de l'huile (2), équipé d'un dispositif (1) servant au moins à mesurer la concentration d'un gaz dissous dans de l'huile isolante (2) de l'équipement électrique (3), comprenant, combinés ensemble :

- un module (10) servant à mesurer les impulsions électriques relatives aux décharges électriques partielles se produisant dans l'équipement électrique (3) et qui génèrent lesdites impulsions ;
- une unité de traitement (12) reliée au dispositif (1) et au module (10) servant à mesurer les décharges partielles et conçue pour déduire au moins un paramètre de concentration corrélé à la concentration de gaz et au moins un paramètre de décharge corrélé aux décharges partielles et pour déduire une indication de diagnostic sur l'état d'isolation de l'équipement électrique (3), en fonction des valeurs dérivées de l'au moins un paramètre de concentration et de l'au moins un paramètre de décharge combinés,

**caractérisé en ce que** l'unité de traitement (12) est configurée pour déduire les paramètres de décharge suivants :

- un indicateur de la présence ou de l'absence de décharges partielles dans l'équipement (3) ;
- une indication de la présence de décharges partielles intermittentes dans l'équipement (3) ; et
- une attribution des décharges partielles mesurées aux catégories interne, surface et couronne,

dans lequel l'unité de traitement (12) est configurée pour traiter les données relatives à un paramètre de phase et à un paramètre d'amplitude des décharges mesurées pour fournir ladite attribution de l'activité de décharge mesurée aux catégories interne, surface et couronne.

16. Appareil selon la revendication 15, comprenant un module d'identification pouvant être relié à une base de données contenant des valeurs de référence d'indicateurs prédéterminés concernant un ensemble de données comprenant au moins lesdits paramètres de concentration et de décharge, lesdites valeurs de référence étant caractéristiques de catégories de source prédéterminées générant les décharges partielles et/ou le gaz dissous dans l'huile, ledit module d'identification étant programmé pour comparer un ensemble de données composé de valeurs dérivées des paramètres de concentration et de décharge avec les données présentes dans la base de données afin d'affecter ledit ensemble de données à une ou plusieurs desdites catégories de source, identifiant ainsi le type de source générant les décharges partielles et/ou le gaz dissous dans l'huile.

17. Appareil selon la revendication 16, dans lequel l'équipement électrique est un transformateur et le module d'identification est adapté pour identifier une ou plusieurs des catégories contenues dans la liste ci-après desdites catégories de sources prédéterminées générant les décharges partielles et/ou le gaz dissous dans l'huile :

- surchauffe du transformateur ;
- production d'arcs au coeur du transformateur ;
- défauts de l'isolation en papier du transformateur ;
- décharges électriques produites dans l'huile par une électrode à haute tension du transformateur ;
- décharges électriques dans des zones faiblement imprégnées à l'intérieur du transformateur ;
- bulles dans l'huile ;
- décharges produites le long d'une surface extérieure de l'isolation du transformateur.

18. Appareil selon l'une quelconque des revendications de 15 à 17, dans lequel le dispositif (1) comprend :

- une membrane (5) perméable au gaz, interposée entre un récipient (7) d'huile (2) et une chambre de mesure (4) qui reçoit une partie du gaz à travers la membrane (5) ;
- un capteur (6) monté dans la chambre de mesure (4) pour mesurer une valeur de la concentration de gaz dans la chambre de mesure (4) ;
- une unité de commande (8) reliée au capteur (6) permettant de déduire une valeur estimée de la concentration de gaz dans l'huile selon la valeur mesurée dans la chambre de mesure (4),

**caractérisé en ce que** l'unité de commande (8) est conçue pour relever, à des instants de mesure successifs, une pluralité de mesures des valeurs de concentration de gaz dans la chambre de mesure (4) et pour calculer la valeur estimée de concentration de gaz dans l'huile, à un instant sélectionné à partir desdits instants de mesure selon une fonction non-linéaire des valeurs mesurées à l'instant de mesure sélectionné et à un ou plusieurs des instants de mesure précédents celui sélectionné.

FIG.1

FIG.2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4654806 A

**Non-patent literature cited in the description**

- **HARRIS D et al.** *conditioning monitoring in power transformers,* 19 June 1997
- **DUVAL M.** the duval triangle for load tap changers, non-mineral oils and low temperature faults in transformers. *IEEE electrical insulation magazine,* 01 November 2008, vol. 11 (6
- **DHINGRA ARVIND et al.** Condition monitoring of power transformer: a review. *Transmission and distribution conference and exposition, T&D IEEE, USA,* 21 April 2008
- **WENZEL D et al.** Partial discharge measurement and gas monitoring of a power transformer on-site. *7th international conference on dielectric materials, measurements and applications, UK,* 23 September 1996